# EUROPEAN PATENT APPLICATION

(11) **EP 0 778 039 A1**
(43) Date of publication of application: **11.06.1997**
(21) Application number: 96119452.9
(22) Date of filing: 04.12.1996
(51) Int. Cl.: A61M 25/01

(54) **Catheter guide wire**

(30) Priority: 07.12.1995 US 568490
(71) Applicant: SARCOS, INC., Salt Lake City Utah 84108 (US)
(72) Inventor: Jacobsen, Stephen C., Salt Lake City, Utah 84192 (US); Davis, Clark, Salt Lake City, Utah 84117 (US); Wells, David, Salt Lake City, Utah 84109 (US)
(74) Representative: Grünecker, Kinkeldey, Stockmair & Schwanhäusser Anwaltssozietät

(57) **Abstract**

A catheter guide wire includes an elongate solid body about which a catheter may be threaded for guidance to a target location in a vasculature passageway of a body. The elongate body includes a proximal end and a distal end, with the distal end being curved. Cuts are formed either by saw-cutting, laser cutting or etching at spaced-apart locations along the length of the body to increase its lateral flexibility, while maintaining its rotational torquability, and to control the direction and degree of flexure.

## Description

### BACKGROUND OF THE INVENTION

This invention relates to catheter guide wire apparatus with improved torque and flexure characteristics.

Catheter guide wires have been used for many years to "lead" or "guide" catheters to desired target locations in the human body's vasculature. The typical guide wire is from about 135 centimeters to 195 centimeters in length, and is made from two primary pieces--a stainless steel core wire, and a platinum alloy coil spring. The core wire is tapered on the distal end to increase its flexibility. The coil spring is typically soldered to the core wire at a point where the inside diameter of the coil spring matches the outside diameter of the core wire. Platinum is selected for the coil spring because it provides radiopacity for X-ray viewing during navigation of the guide wire in the body, and it is biocompatible. The coil spring also provides softness for the tip of the guide wire to reduce the likelihood of puncture of the anatomy.

Navigation through the anatomy is achieved by viewing the guide wire in the body using X-ray fluoroscopy. The guide wire is inserted into a catheter so the guide wire protrudes out the end, and then the wire and catheter are inserted into a vessel or duct and moved therethrough until the guide wire tip reaches a desired vessel or duct branch. The proximal end of the guide wire is then rotated or torqued to point the curved tip into the desired branch and then advanced further. The catheter is advanced over the guide wire to follow or track the wire to the desired location, and provide additional support for the wire. Once the catheter is in place, the guide wire may be withdrawn, depending upon the therapy to be performed. Oftentimes, such as in the case of balloon angioplasty, the guide wire is left in place during the procedure and will be used to exchange catheters.

As the guide wire is advanced into the anatomy, internal resistance from the typically numerous turns, and surface contact, decreases the ability to advance the guide wire further. This, in turn, may lead to a more difficult and prolonged procedure, or, more seriously, failure to access the desired anatomy and thus a failed procedure. A guide wire with both flexibility and good torque characteristics (torsional stiffness) would, of course, help overcome problems created by the internal resistance.

Among the approaches suggested in the prior art for increasing the flexibility of the tip of a guide wire is that of cutting axially spaced grooves in and near the tip, with the depths of the grooves increasing toward the tip. See U.S. Patent No. 5,437,288. The use of cuts to increase flexibility on one side only of a tubular guide wire is disclosed in U.S. Patent No. 5,411,483.

### SUMMARY OF THE INVENTION

It is an object of the invention to provide an improved catheter guide wire apparatus.

It is also an object of the invention to provide such apparatus which exhibits both torsional stiffness, bending flexibility, and longitudinal strength.

It is a further object of the invention to provide such apparatus which is simple in design and construction, for selectively controlling stiffness/flexure of the apparatus.

The above and other objects of the invention are realized in a specific illustrative embodiment of a catheter guide wire apparatus formed of a thin elongate body of material, the exterior surface of which includes a plurality of cuts spaced apart along the length of the body. The cuts extend transversely of the body and are positioned and formed to give the guide wire flexibility without significantly reducing torsional stiffness or strength. One version of this embodiment comprises a solid elongate body with cuts formed alternately on opposite sides of the body, offset from one another to provide desired flexibility without sacrificing strength.

### BRIEF DESCRIPTION OF THE DRAWINGS

The above and other objects, features and advantages of the invention will become apparent from a consideration of the following detailed description presented in connection with the accompanying drawings in which:
FIG. 1 is a side, fragmented view of one embodiment of a catheter guide wire apparatus made in accordance with the principles of the present invention;
FIG. 2 is a side, fragmented view showing two different types of cuts or etchings which may be utilized in a solid guide wire in accordance with the present invention;
FIG. 3 is a side, fragmented view of the tip of a guide wire with radiopaque coil or band wrapped thereabout, in accordance with the present invention;
FIGS. 4 and 5 show side, fragmented views of two embodiments of guide wires formed with cuts, in accordance with the present invention;
FIG. 6 is a side, fragmented view of a tapered guide wire formed with cuts, in accordance with the present invention; and
FIG. 7 is a side, fragmented view of a solid guide wire formed with a coiled tip, in accordance with the present invention.

### DETAILED DESCRIPTION

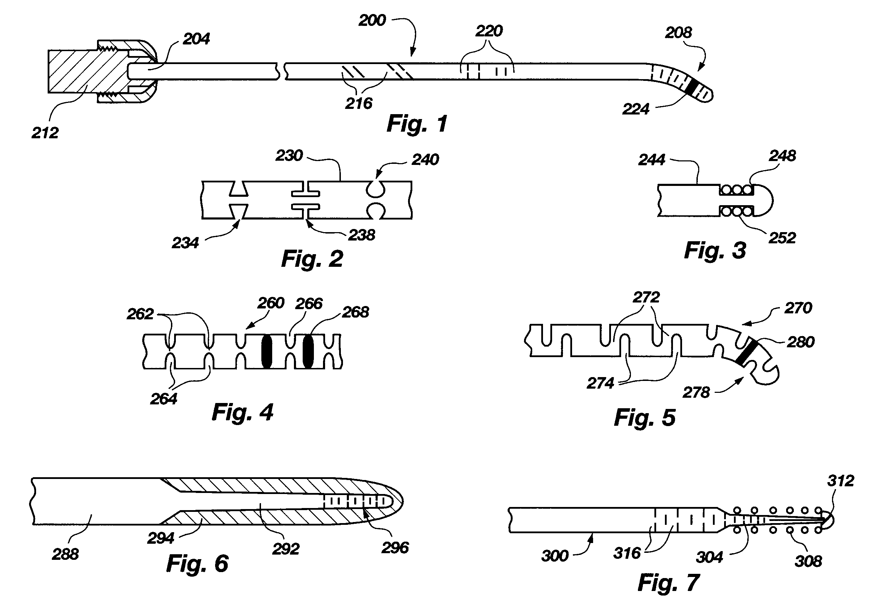

FIG. 1 shows a side, partially cross-sectional, fragmented view of one embodiment of a solid guide wire 200 made in accordance with the present invention. The guide wire 200 includes a proximal end 204 and a distal end 208, with the proximal end being mounted in a conventional pin vise type torquing chuck 212.

Advantageously, the guide wire 200 is constructed of nickel titanium alloy and may range in size from about .008 inches to about .090 inches in diameter and from about 135 to 300 centimeters in length. The guide wire 200 could also be made of stainless steel. Four preferred diameter sizes are .008 inches, .014 inches, .016 inches and .035 inches.

Cuts, slots, gaps or openings 216 and 220 are formed in the guide wire 200 along the length thereof, either by saw cutting (e.g., diamond grit embedded semiconductor dicing blade), etching (for example using the etching process described in U.S Patent No. 5,106,455), laser cutting, or electron discharge machining, to provide for lateral flexibility in the guide wire, while maintaining torsional stiffness. Cuts 216 are angled to allow for a longer cut and thus greater flexibility, whereas the cuts 220 are generally perpendicular to the long dimension of a guide wire. Controlling and varying the spacing, depth and type of cuts allows for selection of the flexure profile of the guide wire, generally the more closely spaced the cuts and the greater depth thereof giving rise to a more flexible guide wire.

The distal end 208 of the guide wire 200 may be preshaped with a curve, as shown, to allow for directing the guide wire around curves and bends. To maintain flexibility in the distal end 208, cuts also be provided on that end. Advantageously, the tip is rounded to minimize the chance of traumatic piercing of body tissue. Also formed on the distal end 208 is a radiopaque marker or band 224. The band 224 may be gold or platinum alloy (for X-ray fluoroscopy) or gadolinium or dysprosium, or compounds thereof (for MRI) and may be formed on the distal end 208 by deposition, wrapping or use of shape memory allow (NiTi) effect to "lock" the band around the end.

FIG. 2 is a side, fragmented view of a guide wire 230, showing three alternative type cuts 234, 238 and 240. These type cuts provide a kind of built in flexure stop to prevent further flexure of the guide wire when the cut openings close to contact one another and prevent further flexure in that direction. The cuts 234 are formed on opposite sides of the guide wire 230 (but could be formed at circumferentially-spaced locations about the guide wire) and are wedge shaped, with the greater width of the wedge being at the bottom of the cut. The cuts 238 are likewise formed on opposite sides of the guide wire 230 (but again could be formed at circumferentially-spaced locations), in the form of T's, with the cross piece of the T being at the bottom of the cut. The cuts 240 are generally circular as shown. Other cut shapes could also be provided to meet the needs of the user. All three type cuts allow for the flexible section (beam) formed by the cuts to be longer than the gap of the flexure stop. This allows the amount of strain in the beam prior to stop engagement to be controlled by varying the ratio of beam length to gap size. This allows for more flexibility, i.e. less bending resistance.

Advantageously, longitudinally adjacent pairs of cuts are rotated about 90 degrees around the wire from one another to provide flexure laterally and vertically. However, the cuts may be located to provide preferential flexure in only one, two, three, etc. directions, if that is desired. Of course, the cuts could be randomly formed to allow bending (flex) equally, non-preferentially in all directions or planes. This could be achieved by circumferentially spacing the cuts.

FIG. 3 shows an alternative embodiment for applying a radiopaque marker to the distal end of a guide wire 244, shown in side, fragmented view. An annular trough or channel 248 is formed at the tip of the guide wire 244, and a radiopaque wire coil, preferably made of platinum alloy, is wound about the guide wire in the channel. The coil 252 could be welded or soldered to itself to hold it in place at the tip of the guide wire 244. If a gold or platinum band is used with a nickel titanium alloy guide wire, the guide wire could be cooled and deformed to allow the coil to be placed on the wire and then when the guide wire were returned to room temperature, the coil would be maintained in place on the guide wire without the need for welding or soldering or other joining mechanism, except for joining the coil to itself.

FIG. 4 is a side, fragmented view of a solid guide wire 260 formed with opposing cuts 264 spaced along a portion of the guide wire, and opposed cuts 266 rotated 90 degrees from opposed cuts 268. Of course, the cuts could be formed to provide preferential bending (flex) in one plane, or could be positioned to allow bending in multiple planes. This could be achieved, for example, by rotating adjacent pairs of cuts by 45 degrees with respect to one another or some other selected amount.

FIG. 5 is a side, fragmented view of a solid guide wire 270 formed with staggered or offset cuts 274 on opposite sides of the guide wire. A curved distal end 278 is also shown with a radiopaque marker band 280. As with the FIG. 4 embodiment, certain pairs of offset cuts could be rotated with respect to the other pairs, to thereby control direction of flexure.

As is evident from FIG. 4, opposed cuts produce thin flexure beams 262 between the bottoms of each pair of opposed cuts. The dimensions and flexure properties of these beams are determined by the depth, separation and width of the cuts and so the flexibility of a guide wire with opposed cuts may be controlled by varying these parameters.

Offset cuts, as indicated in FIG. 5, produce thin flexure beams 272 in the area between each pair of adjacent cuts. The dimensions and flexure properties of these beams are determined not only by the depth and width of the cuts (as with opposed cuts) but also by the offset (axial spacing) of the cuts. Thus the flexibility of a guide wire with offset cuts can be more accurately controlled by varying any or all of these parameters. Also, the flexibility could be varied simply by controlling the degree of the offset while keeping the depth and width of the cuts constant. This provides an advantage for the use of offset cuts since it is more practical to produce a consistent pattern of this type of cut than with opposed cuts. Very flexible sections with opposed cuts require very deep and/or wide cuts, and controlling either parameter may be problematic since very deep cuts could overly weaken the guide wire and very wide cuts may result in catching on and/or damaging tissue through which the guide wire is threaded. Very flexible beams using the offset cut pattern, on the other hand, may be produced without the need for either deep or wide cuts, but rather by simply varying the distance or separation of the offset cuts, and this may be done very accurately.

FIG. 6 is a side, fragmented view of a solid guide wire 284 having an enlarged section 288, which provides more torquability, and a narrowed section 292, covered by a hydrophilic polymer sleeve 294. For example, the enlarged section could be .014 inches in diameter whereas the narrowed section could be .010 inches in diameter. A distal end 296 of the guide wire 284 is formed with cuts as earlier described. Of course, cuts could also be provided at other locations in the narrowed section 292 or in the enlarged section 288, to increase flexibility, while maintaining high torsional stiffness.

FIG. 7 is a side, fragmented view of a solid guide wire 300 having a tapered distal end 304 about which is wrapped a coil 308 made, for example, of platinum alloy. Disposed at the tip of the distal end 304 of the guide wire and in the end of the coil 308 is a solder ball 312. Cuts 316 may also be formed in the guide wire 300 as discussed earlier. In addition to the use of cuts to control the flexure of a guide wire, nickel titanium alloy guide wires can be heat treated to vary the flexure characteristics. For example, selective annealing along the length of the wire can change stress/strain relationship of the material, and thus the flexure.

In the embodiments of a solid guide wire discussed above, the guide wires can be made "flow directable" by providing highly flexible distal ends. "Flow directability" means that the distal end of the guide wire tends to "flow" with the blood around curves and bends in a vasculature passageway. To reduce resistance to movement of a guide wire in a vasculature passageway, the surface of the guide wire may be electropolished to increase the smoothness thereof, and additionally, a lubricious coating may be applied to the surface of the guide wire--such coatings might illustratively include silicone based oil and/or polymer or hydrophilic polymers. Alternatively, a lubricious sleeve made, for example, of a hydrophilic polymer could also be provided for disposal over the guide wire.

It is to be understood that the above-described arrangements are only illustrative of the application of the principles of the present invention. Numerous modifications and alternative arrangements may be devised by those skilled in the art without departing from the spirit and scope of the present invention and the appended claims are intended to cover such modifications and arrangements.

## Claims

1. A catheter guide wire for introduction into a vessel pathway to guide a catheter to a predetermined location, comprising a thin elongate body of material, the exterior surface of which includes a plurality of cuts spaced apart along the length of the body to increase lateral flexibility of the body, said cuts extending crosswise of the body.

2. A catheter guide wire as in Claim 1 wherein the longitudinal spacing between cuts is selectively varied to thereby selectively vary flexibility along the length of the body.

3. A catheter guide wire as in Claim 1 wherein said cuts are formed as successive pairs along the length of the body, with one cut of each pair being formed on the opposite side and longitudinally offset from the other cut of the pair.

4. A catheter guide wire as in Claim 3 wherein at least some of said pairs of cuts are circumferentially rotated some angular amount from the other pairs.

5. A catheter guide wire as in Claim 1 wherein said cuts are formed as successive pairs along the length of the body, with one cut of each pair being formed diagonally opposite the other cut of the pair.

6. A catheter guide wire as in Claim 5 wherein some pairs of cuts are circumferentially rotated some angular amount from other pairs.

7. A catheter guide wire as in Claims 6 wherein alternate pairs of cuts are circumferentially rotated about 90 degrees from adjacent pairs.

8. A catheter guide wire as in Claim 1 wherein said cuts extend at an angle of about ninety degrees with respect to the long axis of the body.

9. A catheter guide wire as in Claim 1 wherein said cuts extend at an acute angle with respect to the long axis of the body.

10. A catheter guide wire as in Claim 1 wherein a first group of said cuts are formed on a top side of the body, a second group on a bottom side, a third group on a first lateral side, and a fourth group on the opposite lateral side, and wherein the groups of cuts are interleaved with one another.

11. A catheter guide wire as in Claim 1 wherein at least some of said cuts are each formed with a wedge-shaped cross-section, with the widest part of the wedge forming the bottom of said each cut.

12. A catheter guide wire as in Claim 1 wherein at least some of said cuts are each formed with a T-shaped cross-section, with the cross-bar portion of the T forming the bottom of said each cut.

13. A catheter guide wire as in Claim 1 wherein at least some of said cuts are each formed with a generally circular cross-section.

14. A catheter guide wire as in Claim 1 wherein said cuts are formed by saw-cutting.

15. A catheter guide wire as in Claim 1 wherein said cuts are formed by etching.

16. A catheter guide wire as in Claim 1 wherein said cuts are formed by laser cutting.

17. A catheter guide wire as in Claim 1 wherein said cuts are formed by electron discharge machining.

18. A catheter guide wire as in Claim 1 wherein the elongate body has a proximal end and distal end, and wherein the distal end is curved.

19. A catheter guide wire as in Claim 1 wherein the elongate body has a proximal end and distal end, and wherein said guide wire further includes a laterally flexible coil mounted on the distal end of the body to project co-linearly outwardly therefrom, to terminate in a leading coil end.

20. A catheter guide wire as in Claim 19 further including a ball disposed in the leading end of the coil.

21. A catheter guide wire as in Claim 1 wherein the elongate body has a proximal end and distal end, and wherein the guide wire further includes a radiopaque element disposed at the distal end of the elongate body.

22. A catheter guide wire as in Claim 21 wherein said radiopaque element comprises a band encircling the distal end of the elongate body.

23. A catheter guide wire as in Claim 21 wherein said radiopaque element comprises a coil wrapped about the distal end of the elongate body.

24. A catheter guide wire as in Claim 23 wherein the distal end of the elongate body is formed with an annular channel circumscribing the body, and wherein said coil is disposed in the channel.

25. A catheter guide wire as in Claim 1 wherein the body of material is solid and generally cylindrical.

26. A catheter guide wire as in Claim 25 wherein the body of material has a proximal end and a distal end, and wherein a portion of the body at the distal end is tapered down to have a smaller diameter than the remaining part of the body.

27. A catheter guide wire as in Claim 26 wherein the smaller diameter is about .010 inches.

28. A catheter guide wire as in Claim 1 wherein the material is selected from the group consisting of nickel-titanium alloy and stainless steel.

29. A catheter guide wire as in Claim 1 further including a lubricious coating disposed over the exterior of the guide wire.

30. A catheter guide wire as in Claim 1 further including a lubricious sleeve disposed about the exterior of the guide wire.
